# EUROPEAN PATENT SPECIFICATION

(11) **EP 4 447 810 B1**
(45) Date of publication and mention of the grant of the patent: **30.04.2025**
(21) Application number: 23822371.3
(22) Date of filing: 13.12.2023
(51) Int. Cl.: A61B 6/00, A61B 6/58

(54) **AUTOMATIC OPTIMAL X-RAY EMITTER POSITION DETECTION FOR MOBILE X-RAY DEVICES**
AUTOMATISCHE OPTIMALE RÖNTGENSTRAHLERPOSITIONSDETEKTION FÜR MOBILE RÖNTGENVORRICHTUNGEN
DÉTECTION AUTOMATIQUE OPTIMALE DE LA POSITION D'UN ÉMETTEUR DE RAYONS X POUR DES DISPOSITIFS MOBILES À RAYONS X

(30) Priority: 22.12.2022 RU 2022133850
(43) Date of publication of application: 23.10.2024
(73) Proprietor: Koninklijke Philips N.V., 5656 AG Eindhoven (NL)
(72) Inventor: SIRAZITDINOV, Ilyas, 5656 AG Eindhoven (NL); STADELMANN, Joël Valentin, 5656 AG Eindhoven (NL)
(74) Representative: Philips Intellectual Property & Standards
(86) International application number: PCT/EP2023/085463
(87) International publication number: WO 2024/132731

(56) References cited:
- US-A1- 2020 085 385
- US-B2- 10 213 623
- US-B2- 11 389 122

## Description

### FIELD OF THE INVENTION

The present invention relates to a computer-implemented method for determining a position of an X-ray emitter of a mobile X-ray device, to an X-ray emitter position determination device, to an X-ray imaging system, to a computer program product, and to a computer-readable medium.

### BACKGROUND OF THE INVENTION

Mobile X-ray, also referred to as portable X-ray, is an important image acquisition modality. It allows to take X-ray in places where stationary X-ray imaging is not practical, e.g., emergency department (ED), intensive care unit (ICU), etc. Mobile X-ray targets mainly patients lying in bed. In contrast to stationary X-ray, acquiring an X-ray image on portable X-ray more challenging since there are more degrees of freedom, than for stationary X-ray image.

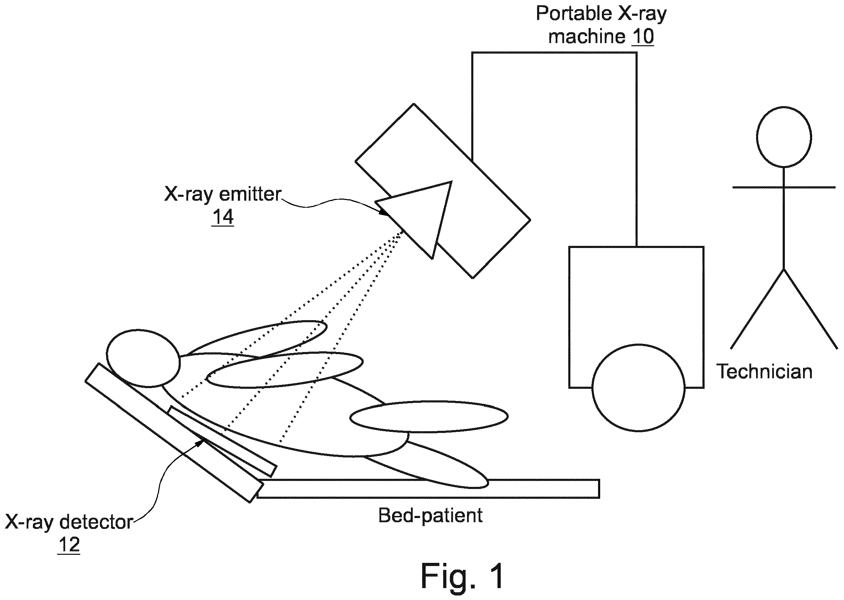

Fig. 1 shows the basic working principle of a mobile X-ray machine 10. First, the technician places an X-ray detector 12 behind a patient, shown as bed-patient in fig. 1. Then, the technician manually sets the position of an X-ray emitter 14 so that the desired field of view can be captured by the X-ray detector 12. Finally, the technician acquires an image by activating the X-ray emitter 14. There are a plenty of parameters that can seriously affect the image quality, such as the position of X-ray detector 12 (e.g., coordinates and rotation angles) and the position of the X-ray emitter (e.g., distance to patient, rotation angles, and voltage).

US2020/0085385Al relates to generating a positioning signal to facilitate positioning of one or more of a patient, X-ray source, or detector during an image acquisition

### SUMMARY OF THE INVENTION

Thus, there may be a need to acquire mobile X-ray images of better quality. The object of the present invention is solved by the subject-matter of the appended independent claims, wherein further embodiments are incorporated in the dependent claims.

According to a first aspect of the present invention, there is provided a computer-implemented method for determining a position of an X-ray emitter of a mobile X-ray device, comprising:
a) receiving (i) a camera-acquired image originating from a camera that monitors a patient in an X-ray imaging session, wherein the camera is registered to an origin of the X-ray emitter, (ii) a reference X-ray image of an internal body structure to be examined in the X-ray examination, and (iii) position information of an X-ray detector;
b) detecting and localizing a plurality of anatomical landmarks in the camera-acquired image;
c) determining a distance from the X-ray emitter to the patient based on the camera-acquired image;
d) generating a virtual X-ray image of the internal body structure based on the plurality of detected anatomical landmarks, the distance from the X-ray emitter to the patient, and the position information of the X-ray detector;
e) registering the virtual X-ray image of the internal body structure with the reference X-ray image of the internal body structure; and
f) determining at least one parameter to adjust the position of the X-ray emitter based on a result of registration.

In other words, the present disclosure proposes a method that can automatically locate the optimal X-ray emitter position for an image using either a reference X-ray image, such as a prior high-quality image from the same patient or a reference image from an atlas, deemed suitable for this imaging case. With the method disclosed herein, a reduction of the radiation dose received by the patients may be achieved by lowering the number of retakes. Additionally, reduced turnaround times may be achieved by avoiding low quality images being send to the PACS and rejected at the time of their review. Furthermore, because of the multiple job repetitions may be reduced, the costs may also be reduced.

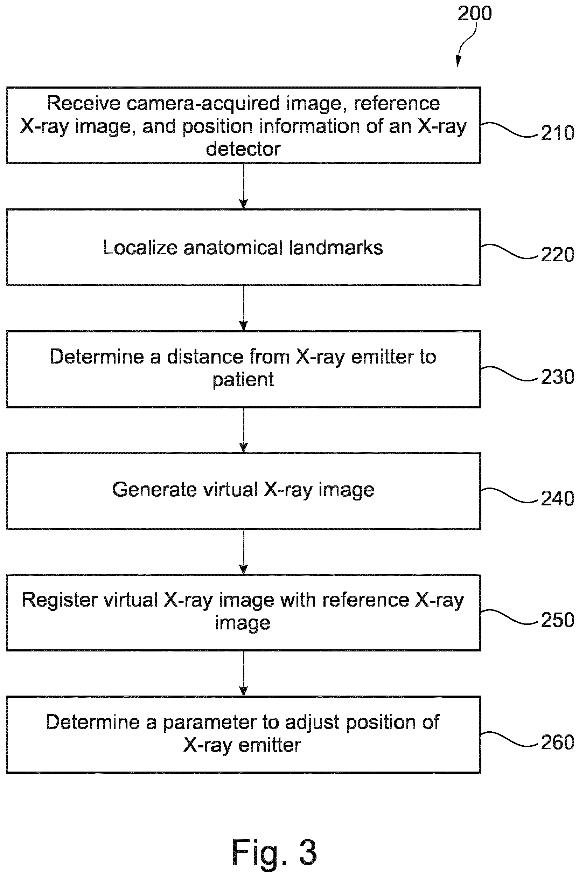

*This will be explained in detail hereinafter and in particular with respect to the examples shown in* *Fig. 3**.*

The step of generating a virtual X-ray image of the internal body structure further comprises:
- producing a pseudo density image of the patient based on the plurality of detected anatomical landmarks; and
- projecting the pseudo density image of the patient to the X-ray detector based on a camera position of the camera, the distance from the X-ray emitter to the patient, and position information of the X-ray detector using a cone-beam projection to obtain the virtual X-ray image of the internal body structure.

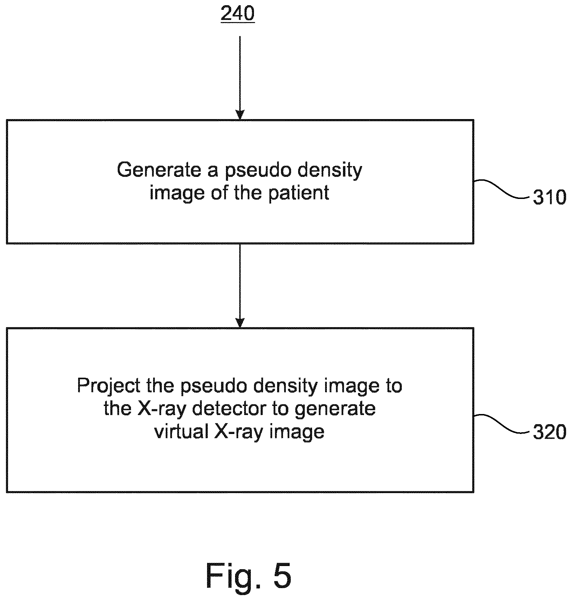

This will be explained in detail hereinafter and in particular with respect to the example shown in Fig. 5.

According to an embodiment of the present invention, the camera comprises a three-dimensional (3D) camera and/or a two-dimensional (2D) camera. The distance from the X-ray emitter to the patient is determined based on depth information in the camera-acquired image or a neural network based depth estimation.

This will be explained in detail hereinafter and in particular with respect to step 230 shown in Fig.. 3.

According to an embodiment of the present invention, the step of registering the virtual X-ray image of the internal body structure with the reference X-ray image of the internal body structure further comprises:
- applying a pre-trained neural network to register the virtual X-ray image of the internal body structure with the reference X-ray image of the internal body structure, wherein the pre-trained neural network has been trained to generate a residual parameter of a camera position of the camera from the virtual X-ray image, the reference X-ray image, and the plurality of detected anatomical landmarks, wherein the residual parameter of the camera position is usable to transform the virtual X-ray image to the reference X-ray image.

This will be explained in detail hereinafter and in particular with respect to the example shown in Fig. 3.

According to an embodiment of the present invention, the at least one parameter for adjusting the position of the X-ray emitter includes one or more of:
- a parameter for adjusting the distance from the X-ray emitter to the patient; and
- a parameter for adjusting a rotation angle of the X-ray emitter.

According to an embodiment of the present invention, the computer-implemented method further comprises:
- providing, based on the at least one determined parameter, an instruction signal to guide a user to manually adjust the position of the X-ray emitter.

According to an embodiment of the present invention, the computer-implemented method further comprises:
- providing, based on the at least one determined parameter, a control signal usable to control a mobile X-ray robotic arm of the mobile X-ray device to adjust the position of the X-ray emitter.

According to an embodiment of the present invention, the reference X-ray image comprises one or more of:
- a patient's previously acquired X-ray image of the internal body structure; and
- a reference X-ray image from an atlas.

According to a second aspect of the present invention, there is provided an X-ray emitter position determination device comprising a processing unit configured to perform the steps of the method according to the first aspect and any associated example.

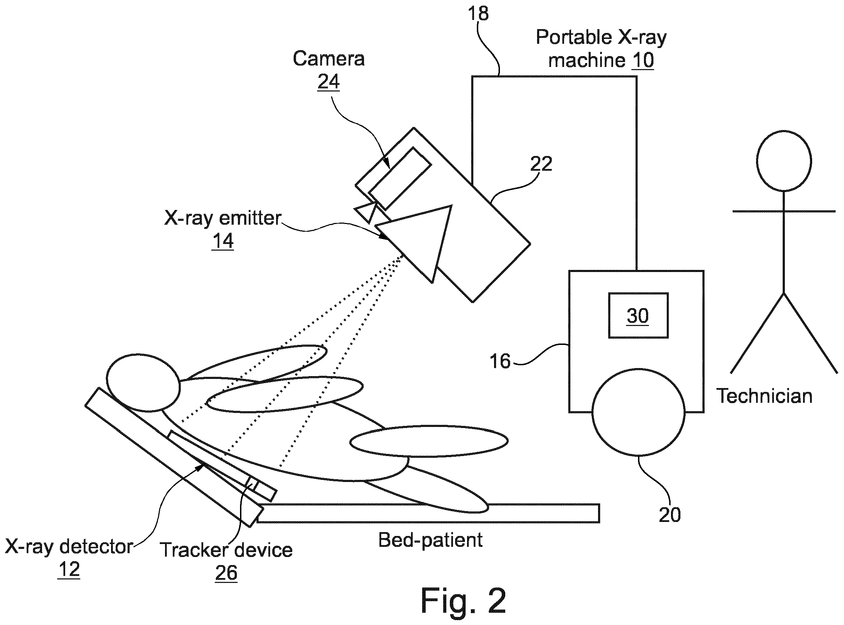

This will be explained in detail hereinafter and in particular with respect to the example shown in Fig. 2.

According to a third aspect of the present invention, there is provided an X-ray imaging system, comprising:
- a mobile X-ray device comprising an X-ray emitter;
- a camera mountable to the mobile X-ray device and configured to capture a camera-acquired image from a patient in an X-ray imaging session, wherein the camera is registered to an origin of the X-ray emitter;
- an X-ray detector;
- a tracker device that is attachable to the X-ray detector or embedded in the X-ray detector, and configured to provide position information of the X-ray detector; and
- the X-ray emitter position determination device according to the second aspect and any associated example configured to determine at least one parameter to adjust a position of the X-ray emitter.

This will be explained in detail hereinafter and in particular with respect to the example shown in Fig. 2.

According to an embodiment of the present invention, the tracker device comprises one or more of: a marker device, a gyroscope, and an antenna.

According to an embodiment of the present invention, the mobile X-ray device further comprises a mobile X-ray robotic arm to support the X-ray emitter. The X-ray emitter position determination device is configured to provide a control signal to control the mobile X-ray robotic arm of the mobile X-ray device to adjust the position of the X-ray emitter.

According to an embodiment of the present invention, the mobile X-ray device further comprises a display configured to display an instruction provided by the X-ray emitter position determination device to guide a user to manually adjust the position of the X-ray emitter.

The predicted directions are used on the X-ray machine: they either can be displayed for the manual correction by the technician, or an automated correction by a mobile X-ray robotic arm (if present) can be realized.

According to another aspect of the present invention, there is provided a computer program product comprising instructions which, when the program is executed by a processing unit, cause the processing unit to carry out the steps of the method disclosed herein.

According to a further aspect of the present invention, there is provided a computer-readable medium having stored thereon the computer program product.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig. 1 shows a basic working principle of a mobile X-ray machine.
Fig. 2 illustrates an exemplary X-ray imaging system.
Fig. 3 illustrates a flowchart describing a computer-implemented method for determining a position of an X-ray emitter of a mobile X-ray device.
Fig. 4 illustrates an exemplary registration process.
Fig. 5 shows a flow diagram describing the generation of a virtual X-ray image of the internal body structure, according to an implementation.
Fig 6 shows an exemplary working principle of the exemplary X-ray imaging system shown in Fig. 2

### DETAILED DESCRIPTION OF EMBODIMENTS

In clinical practice, mobile X-ray systems may be limited in terms of image quality compared to their stationary counterparts, i.e., bad field of view, missed or cut anatomy. Bad images may lead to image retakes, which increase the radiation dose for the patient.

Towards this end, a method, apparatus, and system are provided with the aim of improving the image quality and overcoming one or more of the above-mentioned problems of mobile X-ray during the image acquisition stage. In particular, a system is proposed that can automatically acquire mobile X-ray images of better quality. The system disclosed herein may automatically locate the optimal X-ray emitter position for an image using either a prior high-quality image from the same patient, or a reference image from an atlas, deemed suitable for this imaging case.

Fig. 2 illustrates an exemplary X-ray imaging system 100 according to an embodiment of the present invention. The X-ray imaging system 100 comprises a mobile X-ray device 10 with an X-ray emitter 14. As shown in Fig. 2, the mobile X-ray device 10 further comprises a chassis 16 that supports an arm, e.g., a robotic arm 18, and having a system with wheels 20 for manual or motorized movement which allow the equipment to be transported. The robotic arm 18 may move horizontally and/or vertically and support at its end a head assembly 22, where the X-ray emitter 14 is located.

The X-ray imaging system 100 further comprises a camera 24 mountable to the mobile X-ray device 10 and configured to capture a camera-acquired image from a patient in an X-ray imaging session. The camera 24 is registered to an origin of the X-ray emitter 14. For example, as shown in Fig. 2, the camera 24 may be located in the head assembly 22. In some examples, the camera 24 may be an embedded camera. In some other examples, the camera 24 may be removably mounted to the head assembly 22. In some examples, the camera 24 may be a two-dimensional (2D) camera configured for capturing one scene by using one photographing lens and one image sensor. The obtained image is called a 2D image. In some examples, the camera 24 may be a three-dimensional (3D) camera for capturing 3D images. The 3D camera may be a range camera, which produces a 2D image showing the distance to points in a scene from a specific point. The 3D camera may be e.g., a stereo camera, which is a type of camera with two or more lenses with separate image sensors or film frame for each lens.

The X-ray imaging system 100 shown in Fig. 2 further comprises a portable X-ray detector 12, which may be arranged behind a patient in order to measure the flux, spatial distribution, spectrum, and/or other properties of X-rays. The portable X-ray detector 12 may be fitted with a tracker device 26, such as a gyroscope, antennas, a marker or other devices, which help to localize the portable X-ray detector's position relative to the X-ray emitter 14.

The X-ray imaging system 100 shown in Fig. 2 further comprises an X-ray emitter position determination device 30 configured to determine at least one parameter to adjust a position of the X-ray emitter 14. In general, the X-ray emitter position determination device 30 may comprise various physical and/or logical components for communicating and manipulating information, which may be implemented as hardware components (e.g., computing devices, processors, logic devices), executable computer program instructions (e.g., firmware, software) to be executed by various hardware components, or any combination thereof, as desired for a given set of design parameters or performance constraints.

In some implementations, the X-ray emitter position determination device 30 may be embodied as, or in, a device, such as the mobile X-ray device 10 shown in Fig. 2 or mobile device. The X-ray emitter position determination device 30 may comprise one or more microprocessors or computer processors, which execute appropriate software. The processing unit of the apparatus 10 may be embodied by one or more of these processors. The software may have been downloaded and/or stored in a corresponding memory, e.g., a volatile memory such as RAM or a non-volatile memory such as flash. The software may comprise instructions configuring the one or more processors to perform the functions as described herein.

It is noted that the X-ray emitter position determination device 30 may be implemented with or without employing a processor, and also may be implemented as a combination of dedicated hardware to perform some functions and a processor (e.g., one or more programmed microprocessors and associated circuitry) to perform other functions. For example, the functional units of the X-ray emitter position determination device 30 may be implemented in the device or apparatus in the form of programmable logic, e.g., as a Field-Programmable Gate Array (FPGA). In general, each functional unit of the apparatus may be implemented in the form of a circuit.

Although Fig. 2 may show that the X-ray emitter position determination device 30 is embodied in the mobile X-ray device 10, it will be appreciated that in some implementations the X-ray emitter position determination device 30 may be embodied as, or in, a mobile device, e.g., tablet computer.

The X-ray emitter position determination device 30 is configured to perform the method disclosed herein. The method will be described in detail hereinafter and in conjunction with the flowchart shown in Fig. 3.

Fig. 3 illustrates a flowchart describing a computer-implemented method 200 for determining a position of an X-ray emitter of a mobile X-ray device. The method 200 may be implemented as a device, module or related component in a set of logic instructions stored in a nontransitory machine- or computer-readable storage medium such as random access memory (RAM), read only memory (ROM), programmable ROM (PROM), firmware, flash memory, etc., in configurable logic such as, for example, programmable logic arrays (PLAs), field programmable gate arrays (FPGAs), complex programmable logic devices (CPLDs), in fixed-functionality hardware logic using circuit technology such as, for example, application specific integrated circuit (ASIC), complementary metal oxide semiconductor (CMOS) or transistor-transistor logic (TTL) technology, or any combination thereof. For example, computer program code to carry out operations shown in the method 200 may be written in any combination of one or more programming languages, including an object oriented programming language such as JAVA, SMALLTALK, C++, Python, or the like and conventional procedural programming languages, such as the "C" programming language or similar programming languages. For example, the exemplary method may be implemented as the apparatus 30 shown in Fig. 2.

In step 210, the method 200 comprises a step of receiving (i) a camera-acquired image originating from a camera that monitors a patient in an X-ray imaging session, wherein the camera is registered to an origin of the X-ray emitter, (ii) a reference X-ray image of an internal body structure to be examined in the X-ray examination, and (iii) position information of an X-ray detector.

The camera-acquired image may originate from the camera 24 shown in Fig. 2 that monitors the patient in an X-ray imaging session. In some examples, the camera 24 may be a video camera configured to send the real-time video stream to the apparatus 30, which may have a videoprocessing unit to process the real-time video stream. In some examples, the camera 24 may be a camera configured to capture images and send the images to the apparatus 30, which may have an imageprocessing unit to process the images. In some examples, the camera-acquired image may comprise one or more 3D images. In some examples, the camera-acquired image may comprise one or more 2D images.

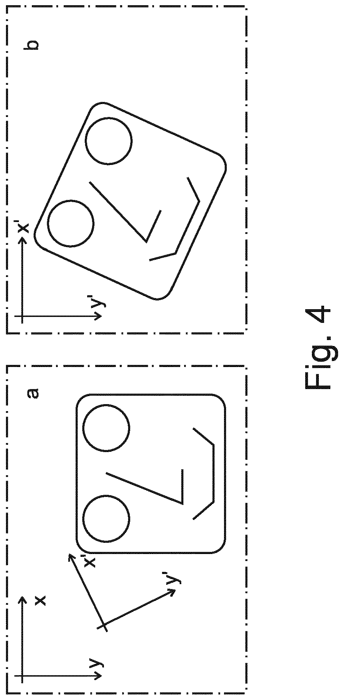

The camera 24 is registered to an origin of the X-ray emitter 14. Fig. 4 illustrates an exemplary registration process. When an object is imaged, its representation is stored in a matrix of pixels, which can be addressed by their coordinates *x, y.* Usually, the origin (i.e., the 0, 0 point) is located in the upper left corner of the matrix, with the x axis going from left to right, and the y axis from top to bottom. Unless the object and the camera are rigidly attached to each other, imaging the object twice will result in two different matrices, with two different coordinate systems. In the example of Fig. 4, the nose of the patient on image "a" is located further on the right and down, than on image "b". It is possible to register the two images together to evaluate the coordinate transformation, which permits to transform one image into the other. Since the imaging planes is perpendicular to the axis of the camera, the transformation of the images directly translates into the camera movements required to reacquire one image if the second is available.

In the present disclosure it is proposed to use image registration in two separated instances:
The transformation from the video camera to the emitter coordinate systems is constant and may be expressed explicitly when designing the relative positions of the two objects; and
The transformation between the positions of the emitter during two different imaging session is likely to be more complicated, than the translation and rotation shown on Fig. 4. Therefore, it is proposed to learn this transformation using a neural network, e.g., a convolutional network. This will be explained in detail hereinafter.

The reference X-ray image of an internal body structure to be examined in the X-ray examination may be downloaded from a Picture Archiving and Communication System (PACS). The reference X-ray image may be a previous scan of the patient of a good quality or some reference image or atlas of a good quality. In some examples, the reference X-ray image may be acquired from a different patient.

The position information of an X-ray detector 12 may be acquired from the tracker device 26, e.g., a marker device, a gyroscope, an antenna, or any combination thereof, which is attached to the X-ray detector 12.

In step 220, the method 200 further comprises a step of detecting and localizing a plurality of anatomical landmarks in the camera-acquired image. In some examples, a model of the patient may be determined prior to imaging in order to conform the imaging parameters to the patient anatomy. The model may include locations of anatomical landmarks, such as shoulders, pelvis, torso, knees, etc. An acquired surface image of a patient may be compared against a library of pre-modeled surface images to determine a model corresponding to the patient. The determination may be performed by a neural network which is trained based on the library of pre-modeled surface images. In some examples, the location of a head, shoulder, torso, knee, and ankle may be determined based on a 2D or 3D image. Neural networks may be trained to detect the landmarks automatically. For example, supervised learning can be applied for anatomical landmark localization. See for example Gite, S., Mishra, A., & Kotecha, K. (2022). Enhanced lung image segmentation using deep learning. Neural Computing and Applications, 1-15*.*

In step 230, the method 200 further comprises a step of determining a distance from the X-ray emitter to the patient based on the camera-acquired image. If the camera is a 3D camera, the distance from the X-ray emitter to the patient may be determined based on depth information in the camera-acquired image. If the camera is a 2D camera, the distance from the X-ray emitter to the patient may be determined based on a neural network based depth estimation. For example, self-supervised learning may be applied for the distance or depth estimation. See for example Bian, J., Li, Z., Wang, N., Zhan, H., Shen, C., Cheng, M. M., & Reid, I. (2019). Unsupervised scale-consistent depth and egomotion learning from monocular video. Advances in neural information processing systems, 32*.*

In step 240, the method 200 further comprises a step of generating a virtual X-ray image of the internal body structure based on the plurality of detected anatomical landmarks, the distance from the X-ray emitter to the patient, and the position information of the X-ray detector.

Fig. 5 shows a flow diagram describing one implementation of step 240.

In step 310 of step 240, a pseudo density image of the patient is generated based on the plurality of detected anatomical landmarks.

In step 320 of step 240, the pseudo density image of the patient is projected to the X-ray detector based on a camera position of the camera, the distance from the X-ray emitter to the patient, and position information of the X-ray detector using a cone-beam projection to obtain the virtual X-ray image of the internal body structure.

In other words, a virtual X-ray image may be generated in two steps. Frist, anatomical landmarks are used to produce a pseudo density image of the patient e.g., by a neural network. Such network can be trained using annotated pairs of images, e.g., CT images and the corresponding masks. The neural network may be a convolutional network. Adversarial training may be used to train this model. See for example Park, T., Liu, M. Y., Wang, T. C., & Zhu, J. Y. (2019). Semantic image synthesis with spatially-adaptive normalization. In Proceedings of the IEEE/CVF conference on computer vision and pattern recognition (pp. 2337-2346*).* The resulted pseudo density image is then projected to the detector given the camera position, distance to patient, and the relative detector position using cone-beam projection. The obtained image is a virtual X-ray image.

Turning back to Fig. 3, in step 250, the method 200 further comprises a step of registering the virtual X-ray image of the internal body structure with the reference X-ray image of the internal body structure
In some examples, a pre-trained neural network may be applied to register the virtual X-ray image of the internal body structure with the reference X-ray image of the internal body structure. The pre-trained neural network has been trained to generate a residual parameter of a camera position of the camera from the virtual X-ray image, the reference X-ray image, and the plurality of detected anatomical landmarks. The residual parameter of the camera position is usable to transform the virtual X-ray image to the reference X-ray image. For example, a neural network may have been trained in the following way. The neural network takes three inputs: a reference X-ray image, a virtual X-ray image, and an anatomical landmarks map. The output of the model is a residual parameter of the camera position. Such parameters can be applied to transform virtual image towards reference image. The required images for training are artificially sampled from pseudo CT projects using static detector parameters and flexible emitter. The registration results are used to predict the directions on how to adjust the emitter's position. The neural network may be a convolutional network. Self-supervised learning can be applied to train this model. See for example Dalca, A. V., Balakrishnan, G., Guttag, J., & Sabuncu, M. R. (2018, September). Unsupervised learning for fast probabilistic diffeomorphic registration. In International Conference on Medical Image Computing and Computer-Assisted Intervention (pp. 729-738). Springer, Cham.

In step 260, the method 200 further comprises a step of determining at least one parameter to adjust the position of the X-ray emitter based on a result of registration. In other words, the registration results are used to predict the directions on how to adjust the emitter's position. The at least one parameter may include one or more of a parameter for adjusting the distance from the X-ray emitter to the patient and a parameter for adjusting a rotation angle of the X-ray emitter.

In some examples, the apparatus 30 may provide, based on the at least one determined parameter, an instruction signal to guide a user to manually adjust the position of the X-ray emitter. In some examples, the instruction signal may be a voice signal for guiding a technician to manually correct the position of the X-ray emitter. In some examples, the instruction signals may be a displayed instruction used to guide a technician to manually correct the position of the X-ray emitter. In some other examples, the apparatus 30 may provide based on the at least one determined parameter, a control signal usable to control a mobile X-ray robotic arm 18 of the mobile X-ray device 10 to adjust the position of the X-ray emitter 14.

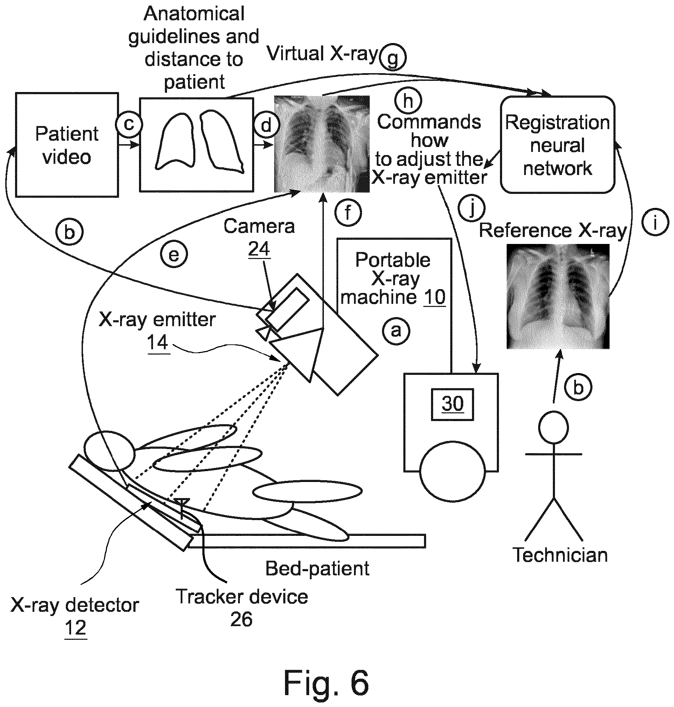

Fig. 6 shows an exemplary working principle of the exemplary X-ray imaging system 100 shown in Fig. 2.

The mobile X-ray machine 10 is placed in front of the patient (indicated with "a"). The X-ray detector 12 is placed behind the patient.

The technician may download from the PACS a reference X-ray image (indicated with "b"). It may be a previous scan of a good quality or some reference image or atlas of a good quality. The reference X-ray image is provided to the apparatus 30. The camera 24 sends a camera-acquired image, e.g., the real-time video stream or images, to the apparatus 30.

The apparatus 30 may comprise a first neural network, also referred to as neural network A, to predict anatomical landmarks and the distance to the patient (indicated with "c") based on the camera-acquired image in the patient video.

The apparatus 30 may comprise a second neural network, also referred to as neural network B, to predict a virtual X-ray image using the anatomical landmarks (indicated with "d") and the X-ray emitter (indicated with "f") and detector position (indicated with "e"). A virtual X-ray image is generated in two steps. 3D anatomical landmarks from neural network A are being used to produce a pseudo density image of the patient by neural network B. Such network can be trained using annotated pairs of images, e.g., CT images and the corresponding masks. The resulted pseudo density image is being projected to the detector given the camera position, distance to patient, and the relative detector position using cone-beam projection. The obtained image is a virtual X-ray image.

The apparatus 30 may comprise a third neural network, also referred to as neural network C, to register the virtual X-ray (indicated with "h") with the reference X-ray image (indicated with "i") using the anatomical landmarks (indicated with "g"). The neural network C has been trained in the following way. It takes three inputs: a reference X-ray image, a virtual X-ray image and an anatomical landmarks map. The output of the model is a residual parameter of the camera position. Such parameters can be applied to transform virtual image towards reference image. The required images for training are artificially sampled from pseudo CT projects using static detector parameters and flexible emitter parameters. The registration results are used to predict the directions on how to adjust the emitter's position.

The predicted directions (indicated with "j") are used on the X-ray machine: they either can be displayed for the manual correction by the technician, or an automated correction by a mobile X-ray robotic arm (if present) can be realized.

The method, apparatus, and system as disclosed herein can automatically locate an optimal X-ray emitter position for an image using either a prior high-quality image from the same patient, or a reference image from an atlas, deemed suitable for this imaging case, and can automatically acquire mobile X-ray images of better quality. The method, apparatus, and system as disclosed herein may lower the number of retakes and therefore achieve a reduction of the radiation dose received by the patients. The method, apparatus, and system as disclosed herein may avoid low quality images being sent to the PACs and rejected at the time of the review and therefore achieve reduced turnaround times. The method, apparatus, and system as disclosed herein may also reduce costs, because of the multiple job repetitions may be reduced.

In another exemplary embodiment of the present invention, a computer program or a computer program element is provided that is characterized by being adapted to execute the method steps of the method according to one of the preceding sfs45kk, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A computer-implemented method for determining a position of an X-ray emitter of a mobile X-ray device, comprising:
- receiving (210) (i) a camera-acquired image originating from a camera that monitors a patient in an X-ray imaging session, wherein the camera is registered to an origin of the X-ray emitter, (ii) a reference X-ray image of an internal body structure to be examined in the X-ray examination, and (iii) position information of an X-ray detector;
- detecting (220) and localizing a plurality of anatomical landmarks in the camera-acquired image;
- determining (230) a distance from the X-ray emitter to the patient based on the camera-acquired image;
**characterised in that**
- generating (240) a virtual X-ray image of the internal body structure based on the plurality of detected anatomical landmarks, the distance from the X-ray emitter to the patient, and the position information of the X-ray detector;
- producing (310) a pseudo density image of the patient based on the plurality of detected anatomical landmarks; and
- projecting (320) the pseudo density image of the patient to the X-ray detector based on a camera position of the camera, the distance from the X-ray emitter to the patient, and position information of the X-ray detector using a cone-beam projection to obtain the virtual X-ray image of the internal body structure,
- registering (250) the virtual X-ray image of the internal body structure with the reference X-ray image of the internal body structure; and
- determining (260) at least one parameter to adjust the position of the X-ray emitter based on a result of registration.

2. The computer-implemented method according to claim 1,
wherein the camera comprises a three-dimensional, 3D, camera and a two-dimensional, 2D, camera; and
wherein the distance from the X-ray emitter to the patient is determined based on depth information in the camera-acquired image or a neural network based depth estimation.

3. The computer-implemented method according to claim 1 or 2,
wherein the step of registering the virtual X-ray image of the internal body structure with the reference X-ray image of the internal body structure further comprises:
- applying a pre-trained neural network to register the virtual X-ray image of the internal body structure with the reference X-ray image of the internal body structure, wherein the pre-trained neural network has been trained to generate a residual parameter of a camera position of the camera from the virtual X-ray image, the reference X-ray image, and the plurality of detected anatomical landmarks, wherein the residual parameter of the camera position is usable to transform the virtual X-ray image to the reference X-ray image.

4. The computer-implemented method according to any one of the preceding claims,
wherein the at least one parameter for adjusting the position of the X-ray emitter includes one or more of:
- a parameter for adjusting the distance from the X-ray emitter to the patient; and
- a parameter for adjusting a rotation angle of the X-ray emitter.

5. The computer-implemented method according to any one of the preceding claims, further comprising:
- providing, based on the at least one determined parameter, an instruction signal to guide a user to manually adjust the position of the X-ray emitter.

6. The computer-implemented method according to any one of the preceding claims, further comprising:
- providing, based on the at least one determined parameter, a control signal usable to control a mobile X-ray robotic arm of the mobile X-ray device to adjust the position of the X-ray emitter.

7. The computer-implemented method according to any one of the preceding claims,
wherein the reference X-ray image comprises one or more of:
- a patient's previously acquired X-ray image of the internal body structure; and
- a reference X-ray image from an atlas.

8. An X-ray emitter position determination device (30) comprising a processing unit configured to perform the steps of the method according to any one of the preceding claims.

9. An X-ray imaging system (100), comprising:
- a mobile X-ray device (10) comprising an X-ray emitter (14);
- a camera (24) mountable to the mobile X-ray device and configured to capture a camera-acquired image from a patient in an X-ray imaging session, wherein the camera is registered to an origin of the X-ray emitter;
- an X-ray detector (12);
- a tracker device (26) that is attachable to the X-ray detector or embedded in the X-ray detector, and configured to provide position information of the X-ray detector; and
- the X-ray emitter position determination device according to claim 8 configured to determine at least one parameter to adjust a position of the X-ray emitter.

10. The X-ray imaging system according to claim 9,
wherein the tracker device comprises one or more of:
- a marker device;
- a gyroscope; and
- an antenna.

11. The X-ray imaging system according to claim 9 or 10,
wherein the mobile X-ray device further comprises a mobile X-ray robotic arm to support the X-ray emitter; and
wherein the X-ray emitter position determination device is configured to provide a control signal to control the mobile X-ray robotic arm of the mobile X-ray device to adjust the position of the X-ray emitter.

12. The X-ray imaging system according to any one of claims 9 to 11, further comprising:
- a display configured to display an instruction provided by the X-ray emitter position determination device to guide a user to manually adjust the position of the X-ray emitter.

13. A computer program product comprising instructions which, when the program is executed by a processing unit, cause the processing unit to carry out the steps of the method according to any one of claims 1 to 7.

14. A computer-readable medium having stored thereon the computer program product of claim 13.

## Patentansprüche

1. Computerimplementiertes Verfahren zum Bestimmen einer Position eines Röntgenstrahlers einer mobilen Röntgenvorrichtung, umfassend:
- Empfangen (210) (i) eines von einer Kamera erfassten Bildes, das von einer Kamera stammt, die einen Patienten in einer Röntgenbildgebungssitzung überwacht, wobei die Kamera auf einen Ursprung des Röntgenstrahlers registriert ist, (ii) eines Referenz-Röntgenbildes einer bei der Röntgenuntersuchung zu untersuchenden inneren Körperstruktur, und (iii) von Positionsinformationen eines Röntgendetektors;
- Detektieren (220) und Lokalisieren einer Vielzahl von anatomischen Orientierungspunkten in dem von der Kamera erfassten Bild;
- Bestimmen (230) eines Abstands vom Röntgenstrahler zum Patienten basierend auf dem von der Kamera erfassten Bild;
**gekennzeichnet durch**
- Generieren (240) eines virtuellen Röntgenbildes der inneren Körperstruktur basierend auf der Vielzahl von detektierten anatomischen Orientierungspunkten, dem Abstand vom Röntgenstrahler zum Patienten und den Positionsinformationen des Röntgendetektors;
- Erzeugen (310) eines Pseudodichtebildes des Patienten basierend auf der Vielzahl von detektierten anatomischen Orientierungspunkten; und
- Projizieren (320) des Pseudodichtebildes des Patienten auf den Röntgendetektor basierend auf einer Kameraposition der Kamera, dem Abstand vom Röntgenstrahler zum Patienten und von Positionsinformationen des Röntgendetektors unter Verwendung einer Kegelstrahlprojektion, um das virtuelle Röntgenbild der inneren Körperstruktur zu erhalten,
- Registrieren (250) des virtuellen Röntgenbildes der inneren Körperstruktur mit dem Referenz-Röntgenbild der inneren Körperstruktur; und
- Bestimmen (260) mindestens eines Parameters, um die Position des Röntgenstrahlers basierend auf einem Registrierungsergebnis anzupassen.

2. Computerimplementiertes Verfahren nach Anspruch 1,
wobei die Kamera eine dreidimensionale, 3D-Kamera und eine zweidimensionale, 2D-Kamera umfasst; und
wobei der Abstand vom Röntgenstrahler zum Patienten basierend auf Tiefeninformationen in dem von der Kamera erfassten Bild oder einer auf einem neuronalen Netzwerk basierenden Tiefenschätzung bestimmt wird.

3. Computerimplementiertes Verfahren nach Anspruch 1 oder 2,
wobei der Schritt des Registrierens des virtuellen Röntgenbildes der inneren Körperstruktur mit dem Referenz-Röntgenbild der inneren Körperstruktur weiter umfasst:
- Anwenden eines vorab trainierten neuronalen Netzwerks, um das virtuelle Röntgenbild der inneren Körperstruktur mit dem Referenz-Röntgenbild der inneren Körperstruktur zu registrieren, wobei das vorab trainierte neuronale Netzwerk trainiert worden ist, um einen Restparameter einer Kameraposition der Kamera aus dem virtuellen Röntgenbild, dem Referenz-Röntgenbild und der Vielzahl von detektierten anatomischen Orientierungspunkten zu generieren, wobei der Restparameter der Kameraposition verwendbar ist, um das virtuelle Röntgenbild in das Referenz-Röntgenbild umzuwandeln.

4. Computerimplementiertes Verfahren nach einem der vorstehenden Ansprüche,
wobei der mindestens eine Parameter zum Anpassen der Position des Röntgenstrahlers eines oder mehr beinhaltet von:
- einem Parameter zum Anpassen des Abstands vom Röntgenstrahler zum Patienten; und
- einem Parameter zum Anpassen eines Drehwinkels des Röntgenstrahlers.

5. Computerimplementiertes Verfahren nach einem der vorstehenden Ansprüche, weiter umfassend:
- Bereitstellen, basierend auf dem mindestens einen bestimmten Parameter, eines Anweisungssignals, um einen Benutzer zu führen, um die Position des Röntgenstrahlers manuell anzupassen.

6. Computerimplementiertes Verfahren nach einem der vorstehenden Ansprüche, weiter umfassend:
- Bereitstellen, basierend auf dem mindestens einen bestimmten Parameter, eines Steuersignals, das verwendbar ist, um einen mobilen Röntgenroboterarm der mobilen Röntgenvorrichtung zu steuern, um die Position des Röntgenstrahlers anzupassen.

7. Computerimplementiertes Verfahren nach einem der vorstehenden Ansprüche, wobei das Referenz-Röntgenbild eines oder mehr umfasst von:
- einem zuvor vom Patienten erfassten Röntgenbild der inneren Körperstruktur; und
- einem Referenz-Röntgenbild aus einem Atlas.

8. Röntgenstrahler-Positionsbestimmungsvorrichtung (30), die eine Verarbeitungseinheit umfasst, die konfiguriert ist, um die Schritte des Verfahrens nach einem der vorstehenden Ansprüche durchzuführen.

9. Röntgenbildgebungssystem (100), umfassend:
- eine mobile Röntgenvorrichtung (10) die einen Röntgenstrahler (14) umfasst;
- eine Kamera (24), die an der mobilen Röntgenvorrichtung montierbar ist und konfiguriert ist, um in einer Röntgenbildgebungssitzung ein von einer Kamera erfasstes Bild eines Patienten aufzunehmen, wobei die Kamera auf einen Ursprung des Röntgenstrahlers registriert ist;
- einen Röntgendetektor (12);
- eine Trackervorrichtung (26), die an dem Röntgendetektor anbringbar, oder in den Röntgendetektor eingebettet ist und konfiguriert ist, um Positionsinformationen des Röntgendetektors bereitzustellen; und
- die Röntgenstrahler-Positionsbestimmungsvorrichtung nach Anspruch 8, die konfiguriert ist, um mindestens einen Parameter zu bestimmen, um eine Position des Röntgenstrahlers anzupassen.

10. Röntgenbildgebungssystem nach Anspruch 9,
wobei die Trackervorrichtung eines oder mehr umfasst von:
- einer Markierungsvorrichtung;
- einem Gyroskop; und
- einer Antenne.

11. Röntgenbildgebungssystem nach Anspruch 9 oder 10,
wobei die mobile Röntgenvorrichtung weiter einen mobilen Röntgenroboterarm umfasst, um den Röntgenstrahler zu tragen; und
wobei die Röntgenstrahler-Positionsbestimmungsvorrichtung konfiguriert ist, um ein Steuersignal bereitzustellen, um den mobilen Röntgenroboterarm der mobilen Röntgenvorrichtung zu steuern, um die Position des Röntgenstrahlers anzupassen.

12. Röntgenbildgebungssystem nach einem der Ansprüche 9 bis 11. weiter umfassend:
- eine Anzeige, die konfiguriert ist, um eine von der Positionsbestimmungsvorrichtung eines Röntgenstrahlers bereitgestellte Anweisung anzuzeigen, um einen Benutzer zu führen, um die Position des Röntgenstrahlers manuell anzupassen.

13. Computerprogrammprodukt, das Anweisungen umfasst, die, wenn das Programm von einer Verarbeitungseinheit ausgeführt wird, die Verarbeitungseinheit veranlassen, die Schritte des Verfahrens nach einem der Ansprüche 1 bis 7 auszuführen.

14. Computerlesbares Medium, auf dem das Computerprogrammprodukt nach Anspruch 13 gespeichert ist.

## Revendications

1. Procédé mis en œuvre par ordinateur pour déterminer une position d'un émetteur de rayons X d'un dispositif à rayons X mobile, comprenant :
- la réception (210) (i) d'une image acquise par caméra provenant d'une caméra qui surveille un patient dans une session d'imagerie à rayons X, dans lequel la caméra est alignée sur une origine de l'émetteur de rayons X, (ii) d'une image radiologique de référence d'une structure de corps interne à examiner dans l'examen radiologique, et (iii) d'informations de position d'un détecteur de rayons X ;
- la détection (220) et la localisation d'une pluralité de repères anatomiques dans l'image acquise par caméra ;
- la détermination (230) d'une distance entre l'émetteur de rayons X et le patient sur la base de l'image acquise par caméra ;
**caractérisé en ce que**
- la génération (240) d'une image radiologique virtuelle de la structure de corps interne sur la base de la pluralité de repères anatomiques détectés, de la distance entre l'émetteur de rayons X et le patient et des informations de position du détecteur de rayons X ;
- la production (310) d'une image de pseudo-densité du patient sur la base de la pluralité de repères anatomiques détectés ; et
- la projection (320) de l'image de pseudo-densité du patient sur le détecteur de rayons X en fonction d'une position de caméra de la caméra, de la distance entre l'émetteur de rayons X et le patient et des informations de position du détecteur de rayons X à l'aide d'une projection à faisceau conique pour obtenir l'image radiologique virtuelle de la structure de corps interne,
- l'alignement (250) de l'image radiologique virtuelle de la structure de corps interne avec l'image radiologique de référence de la structure de corps interne ; et
- la détermination (260) d'au moins un paramètre pour régler la position de l'émetteur de rayons X sur la base d'un résultat d'alignement.

2. Procédé mis en œuvre par ordinateur selon la revendication 1,
dans lequel la caméra comprend une caméra tridimensionnelle, 3D, et une caméra bidimensionnelle, 2D ; et
dans lequel la distance entre l'émetteur de rayons X et le patient est déterminée sur la base d'informations de profondeur dans l'image acquise par caméra ou d'une estimation de profondeur basée sur un réseau neuronal.

3. Procédé mis en œuvre par ordinateur selon la revendication 1 ou 2,
dans lequel l'étape d'alignement de l'image radiologique virtuelle de la structure de corps interne avec l'image radiologique de référence de la structure de corps interne comprend en outre :
- l'application d'un réseau neuronal pré-entraîné pour aligner l'image radiologique virtuelle de la structure de corps interne avec l'image radiologique de référence de la structure de corps interne, dans lequel le réseau neuronal pré-entraîné a été formé pour générer un paramètre résiduel d'une position de caméra de la caméra à partir de l'image radiologique virtuelle, de l'image radiologique de référence et de la pluralité de repères anatomiques détectés, dans lequel le paramètre résiduel de la position de caméra est utilisable pour transformer l'image radiologique virtuelle en image radiologique de référence.

4. Procédé mis en œuvre par ordinateur selon l'une quelconque des revendications précédentes,
dans lequel le au moins un paramètre permettant de régler la position de l'émetteur de rayons X inclut un ou plusieurs des éléments suivants :
- un paramètre permettant de régler la distance entre l'émetteur de rayons X et le patient ; et
- un paramètre permettant de régler un angle de rotation de l'émetteur de rayons X.

5. Procédé mis en œuvre par ordinateur selon l'une quelconque des revendications précédentes, comprenant en outre :
- la fourniture, sur la base du au moins un paramètre déterminé, d'un signal d'instruction pour guider un utilisateur afin de régler manuellement la position de l'émetteur de rayons X.

6. Procédé mis en œuvre par ordinateur selon l'une quelconque des revendications précédentes, comprenant en outre :
- la fourniture, sur la base du au moins un paramètre déterminé, d'un signal de commande utilisable pour commander un bras robotique à rayons X mobile du dispositif à rayons X mobile pour régler la position de l'émetteur de rayons X.

7. Procédé mis en œuvre par ordinateur selon l'une quelconque des revendications précédentes,
dans lequel l'image radiologique de référence comprend un ou plusieurs des éléments suivants :
- une image radiologique du patient précédemment acquise de la structure de corps interne ; et
- une image radiologique de référence provenant d'un atlas.

8. Dispositif de détermination de position d'émetteur de rayons X (30) comprenant une unité de traitement configurée pour exécuter les étapes du procédé selon l'une quelconque des revendications précédentes.

9. Système d'imagerie à rayons X (100) comprenant :
- un dispositif à rayons X mobile (10) comprenant un émetteur de rayons X (14) ;
- une caméra (24) pouvant être montée sur le dispositif à rayons X mobile et configurée pour capturer une image acquise par caméra à partir d'un patient lors d'une session d'imagerie à rayons X, dans lequel la caméra est alignée sur une origine de l'émetteur de rayons X ;
- un détecteur de rayons X (12) ;
- un dispositif de suivi (26) qui peut être fixé au détecteur de rayons X ou intégré dans le détecteur de rayons X et configuré pour fournir des informations de position du détecteur de rayons X ; et
- le dispositif de détermination de position d'émetteur de rayons X selon la revendication 8 configuré pour déterminer au moins un paramètre pour régler une position de l'émetteur de rayons X.

10. Système d'imagerie à rayons X selon la revendication 9,
dans lequel le dispositif de suivi comprend un ou plusieurs éléments parmi :
- un dispositif marqueur ;
- un gyroscope ; et
- une antenne.

11. Système d'imagerie à rayons X selon la revendication 9 ou 10,
dans lequel le dispositif à rayons X mobile comprend en outre un bras robotique à rayons X mobile pour supporter l'émetteur de rayons X ; et
dans lequel le dispositif de détermination de position d'émetteur de rayons X est configuré pour fournir un signal de commande pour commander le bras robotique à rayons X mobile du dispositif à rayons X mobile pour régler la position de l'émetteur de rayons X.

12. Système d'imagerie à rayons X selon l'une quelconque des revendications 9 à 11, comprenant en outre :
- un dispositif d'affichage configuré pour afficher une instruction fournie par le dispositif de détermination de position d'émetteur de rayons X pour guider un utilisateur afin de régler manuellement la position de l'émetteur de rayons X.

13. Produit de programme informatique comprenant des instructions qui, lorsque le programme est exécuté par une unité de traitement, amènent l'unité de traitement à réaliser les étapes du procédé selon l'une quelconque des revendications 1 à 7.

14. Support lisible par ordinateur sur lequel est stocké le produit programme informatique selon la revendication 13.
